# EUROPEAN PATENT SPECIFICATION

(11) **EP 1 330 272 B1**
(45) Date of publication and mention of the grant of the patent: **08.06.2005**
(21) Application number: 01992589.0
(22) Date of filing: 25.10.2001
(51) Int. Cl.: A61L 15/56, A61L 15/58

(54) **HOT MELT WETNESS INDICATOR ADHESIVE COMPOSITION**
HEISSSCHMELZKLEBEZUSAMMENSETZUNG MIT NÄSSEINDIKATOR
COMPOSITION ADHESIVE THERMOFUSIBLE INDICATRICE D'HUMIDITE

(30) Priority: 30.10.2000 US 703374
(43) Date of publication of application: 30.07.2003
(73) Proprietor: Ato Findley Inc, Wauwatosa Wisconsin 53226-3434 (US)
(72) Inventor: WANG, Baoyu, Waukesha, WI 53186 (US)
(74) Representative: Gillam, Francis Cyril
(86) International application number: PCT/US2001/045486
(87) International publication number: WO 2002/036177

(56) References cited:
- US-A- 5 035 691
- US-A- 5 066 711

## Description

### FIELD OF THE INVENTION

The invention relates to a hot melt wetness indicator adhesive composition that can be used in disposable nonwoven absorbent articles. The indicator adhesive will indicate wetness when body fluid is discharge from the wearer. This adhesive can be formulated to have different rates of color change, ranging from an almost instantaneous response, to a delayed color change of a few hours.

### BACKGROUND OF THE INVENTION

Disposable nonwoven absorbent products have widespread acceptance for infant, young child and incontinent adult care applications. Typical disposable nonwoven absorbent articles include diapers, training pants, adult incontinent pads and briefs, feminine sanitary napkins or pads and tampons. Disposable nonwoven absorbent articles such as those, function to receive and contain urine and other body fluids that the wearer secretes. These items are worn against or in close proximity to the skin of the wearer.

Typical disposable nonwoven absorbent articles consist of a fluid-impervious film back sheet, a porous fluid permeable nonwoven top sheet and an absorbent core sandwiched between the top and back sheets. These substrates are usually bonded together using hot melt adhesives. In addition to this basic construction, these absorbent articles usually have many other features to either improve the body fluid containment function or to enhance the comfort level for the wearer. For example, infant diapers contain elastic leg cuffs attached to the top sheet for enhanced fluid containment.

Since disposable nonwoven absorbent articles are widely used for body fluid containment function, it is desirable to know if the device gets wet and requires replacement. Monitoring of wetness by visual inspection can be time consuming and unpleasant. It is therefore invaluable to incorporate a function to signal wetness into a disposable nonwoven absorbent article such as a diaper.

Approaches using coating stripes of wetness indicator adhesives, or wetness indicators to signal wetness by way of color change have been described in Mroz et al., U.S. Patent No. 4,231,370. This article discloses an improved absorbent product having a wetness indicator disposed between a translucent cover member and an absorbent member. According to the disclosure, the wetness indictor is applied in the form of a stripe to a portion of the inwardly facing surface of a back sheet of a disposable diaper. Such a wetness indictor contains a pH-change/color-change type of colorant dispersed in a water-based adhesive latex of styrene/2-ethylhexylacrylate copolymer, ethylene/vinyl acetate copolymer, or polyvinyl acetate. The indicator adheres to the back sheet and dries to a flexible coating that is yellow in color. When contacted or "insulted" by body secretions such as urine, the indicator changes from yellow to blue, signaling the presence of moisture. To obtain a suitable pH, sufficient acid buffering means such as phosphoric acid must be added to the latex. Phosphoric acid is a harsh acid, which could raise safety concerns. Another disadvantage of the Mroz et al. latex based composition and any similar water or solvent based products is that water or solvent removal must be provided during manufacturing.

Colon et al. U.S. Patent Nos. 4,681,576, 4,743,238 and 4,895,567 disclose hot melt wetness indicator adhesives which change color upon insult with urine or water. These adhesives are based on a water-soluble polyvinyl pyrrolidone polymer, or a water soluble vinyl pyrrolidone-vinyl acetate copolymer, or an ethylene-acrylic acid copolymer in combination with a fatty acid and a wetness indicating dye. The composition can contain a variety of other ingredients such as water-soluble waxes, glycerol esters, ethylene-vinyl acetate copolymers and hydrogenated oils, etc.

Zimmel et al., U.S. Patent No 5,035,691 discloses a hot melt wetness indicator composition based on an adduct which is prepared by reacting ethylene-acrylic acid copolymer with polyethylene oxide using a monobutyl tin (IV) oxide catalyst. The composition contains 0.03 to 0.5 wt% acid-base indicator as the active ingredient to signal the presence of moisture.

Raykovitz, U.S. Patent No. 5,342,861 discloses a composition similar to that of Zimmel et al. in that the composition consists of a wetness indicating agent such as a pH indicator, a graft copolymer prepared by reacting a vinyl polymer with low molecular weight polyethylene oxide and a compatible tackifier.

The prior art compositions herein mentioned above have several deficiencies. The hot melt wetness indicator composition disclosed in Colon et al., for example, exhibit poor thermal stability. Thus, when heated at elevated temperatures between 121-148° C (250-300°F) which is typically encountered during hot melt application, the adhesives can severely degrade as manifested by char, skin formulation and color darkening. Most of the components in Colon's composition are incompatible with each other, and therefore, the composition can suffer from phase separation during application at the typical hot melt adhesive coating conditions. Other deficiencies are the poor environmental stability and poor bleed-through or wash-out resistance that typical polyvinylpyrrolidone homo- or co-polymer based formulations suffer from after the indicator is applied to a typical polymer film substrate. The coated indicator tends to change slowly and prematurely from yellow to green and finally to blue from exposure to atmospheric moisture during storage. This aspect is particularly important since finished nonwoven adsorbent products can be stored for several months before they reach consumer's hands. A premature color change during storage will render the product useless. An additional deficiency is the poor intensity of color change of the indicator so that in many cases the color change is barely visible through translucent substrates. The compositions taught by Zimmel et al. and Raykovitz, on the the other hand, necessitates harsh conditions to carry out chemical grafting of low molecular weight hydrophilic PEG to another relatively high molecular weight hydrophobic polymer. Their grafting reactions require either an organotin catalyst (Zimmel et al.) or a peroxide initiator (Raykovitz). Problems can arise from product safety concerns with residual organotin compound and peroxides. Since the hydrophilic PEG are typically incompatible with the vinyl polymer used for preparation of graft copolymer, the unreacted reactants can pose compatibility problems for the final wetness indicator composition.

In view of the deficiencies of the prior art products, a need exists for a new hot melt wetness indicator that is compatible, is thermally and environmentally stable, has intense color change and good wash-out resistance, can withstand multiple insults during use, and is easy to manufacture and apply. It has been discovered that a hot melt wetness indicator composition based on ethylene-alkylacrylate-acrylic acid terpolymer can meet all the herein above mentioned requirements.

### SUMMARY OF THE INVENTION

The present invention is directed towards a hot melt wetness indicator composition based on an ethylene-alkylacrylate-acrylic acid terpolymer. The wetness indicator composition comprises the herein mentioned terpolymer, a tackifying resin, a compatible surfactant having a HLB value less than 20 and a pH indicator or a dye that is capable of changing color when insulted with urine as the primary ingredients. The composition of the present invention has overcome the deficiencies of the prior art wetness indicators.

One embodiment of the present invention is to provide a wetness indictor that has excellent heat and environmental stability, improved fastness, vivid color change, easy manufacturing, and easy application. Another embodiment is directed towards a composition having a delayed response and therefore can withstand multiple insults daily.

The hot melt wetness indicator composition of the present invention can be applied using a variety of conventional coating techniques known in the art. It is especially suited for slot die, multibeads, spiral spray and different variations of melt-blown coatings.

The hot melt wetness indicator adhesive composition of the present invention comprises as components thereof a mixture of the following components:
a. an ethylene-alkylacrylate-acrylic acid terpolymer in the amounts of 10% to 40% by weight, and preferably in the range of 15 - 30% by weight;
b. a polar tackifier in the amounts of 20% to 60% by weight, and preferably in the amounts of 25% to 50% by weight;
c. a surfactant or a blend of surfactants in the amount of 15% to 60% by weight, and preferably in the amount of 25 - 45% by weight;
d. a plasticizer in the amount of 0 - 20% by weight, and preferably in the amounts of 5% to 15% by weight;
e. 0 - 2% by weight of a stabilizer or antioxidant; and
f. a pH indicator or acid-base indicator in the amount of 0.01 to 0.5% by weight, and preferably in the amount of 0.05% to 0.2% by weight.

The components of the composition add up to 100%. The adhesive may contain other ingredients such as inert dye that can modify the color of the above basic adhesive composition.

### DETAILED DESCRIPTION OF THE INVENTION

In accordance with the present invention, a hot melt wetness indicator is formulated, comprising as the primary polymer component 10 - 40% by weight an ethylene-alkylacrylate-acrylic acid terpolymer, 20 - 60% by weight of a compatible tackifier, 15 - 60% by weight of a surfactant, 0.01 to 0.5% by weight of a pH indicator, 0 - 20% by weight of a plasticizer and 0 - 2% by weight of a stabilizer or antioxidant. Optional ingredients can be added to modify or enhance the physical and performance characteristics of the composition. Such optional ingredients include, but not limited to, fillers, inert dye stuff or colorant, fluorescing agents, waxes, etc. Other polymers can be added as long as they do not materially affect the properties of the finished adhesive.

The hot melt adhesive composition of the present invention includes an ethylene-alkylacrylate-acrylic acid terpolmyer in the amounts of 10% to 40% by weight, and preferably in the amounts of 15% to 35% by weight. Suitable terpolymers can be obtained by copolymerization of ethylene, acrylic acid and an ester of acrylic or methacrylic acid having the following molecular structure:

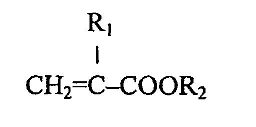

Where R₁ is either a hydrogen (H) or a methyl group (CH₃) and R₂ is either a hydrogen (H) or an alkyl moiety with 1 - 10 carbon chain length. Examples of such esters include methyl acrylate, ethyl acrylate, butyl acrylate, 2-hydroxyl ethyl acrylate, methyl methacrylate, ethyl methacrylate, butyl methacrylate and 2-hydroxy ethyl methacrylate, etc.

The preferred polymers are ethylene-methylacrylate-acrylic acid terpolymers having 5 - 35% by weight methyl acrylate, preferably 10 - 35% by weight methyl acrylate and 2 - 25% by weight acrylic acid, preferably 2 - 15% by weight acrylic acid. The terpolymers should also preferably have a melt index value greater than 5g/10min. The terpolymers of the type described above are commercially available from Exxon Chemical Company under the trade name designation Escor®.

The polar tackifying resins which are used in the hot melt adhesives of the present invention are those which extend adhesive properties and improve specific adhesion. As used herein, the term "polar tackifying resin" include:
(a) natural and modified rosin such as, for example, gum rosin, wood rosin, tall-oil rosin, distilled rosin, hydrogenated rosin, dimerized rosin and polymerized rosin;
(b) glycerol and pentaerythritol esters of natural and modified rosin such as, for example, the glycerol ester of pale wood rosin, the glycerol ester of hydrogenated rosin, the glycerol ester of polymerized rosin, the pentaerythritol ester of pale wood rosin, the pentaerythritol ester of hydrogenated rosin, the pentaerythritol ester of tall-oil rosin, and the phenolic modified pentaerythritol ester of rosin;
(c) phenol-modified terpene resins such as, for example, the resin product resulting from the condensation in an acidic medium of a terpene and a phenol;

Mixtures of two or more of the above described tackifying resins may be used for some formulations. Although tackifiers may be used in the amounts of 20% to 60%, the preferred range is 25% to 50% by weight.

The wetness indicator composition of the present invention contains from 15 - 60% by weight, and preferably 25% to 45% by weight, of a surfactant to impart water permeability to the composition. The surfactants suitable for use herein comprise cationic, anionic or non-ionic types having a HLB value less than 20 with the non-ionic type preferred. The more preferred surfactant is selected from a group of non-ionic surfactants having HLB less than 15. These surfactants include alkyl amines and amides; alkanolamines and amides; amine oxides; ethoxylated fatty alcohols, ethoxylated fatty acids, ethoxylated alkylphenols, ethoxylated amines or amides; ethoxylated fatty esters and oils; glycerol fatty esters and their ethoxylated derivatives; sorbitan derivatives; sucrose and glucose esters and their derivatives. The most preferred surfactants will have a HLB between 3 and 12 and are selected from a subgroup including ethoxylated fatty alcohols, ethoxylated fatty acids, glycerol esters of fatty acids and their derivatives and sorbitan derivatives. Mixtures of two or more surfactants herein described above may be used for some formulations.

A plasticizer may be present in the composition of the present invention in the amounts of 0 - 20% by weight, and preferably in the amounts of 5% to 15% by weight, to provide desired viscosity control. A suitable plasticizer may be selected from dibenzoate esters of a glycol such as diethylene glycol dibenzoate, dipropylene glycol dibenzoate, pentaerythritol terabenzoate, alkyl phthalates such as dibutyl and dioctyl phthalate, alkyl phosphates such as 2-ethylhexyl diphenyl phosphates; fatty esters of polyglycol such as polyethylene glycol 400-di-2 ethyl hexoate; low molecular weight liquid polyethylene and propylene glycols; and low molecular weight liquid copolyesters. Suitable dipropylene glycol dibenzoate and pentaerythritol tetrabenzoate may be purchased from Velsicol Chemical Company of Chicago, IL. under the trade name designations "Benzoflex 9-88 and S-522" respectively. Further, a suitable polyethylene glycol 400-di-2-ethylhexoate may be purchased from C.P. Hall Company of Chicago. IL. under the trade name designation "Tegmer 809". A suitable liquid polyethylene glycol may be obtained from Union Carbide Chemical Company under the trade name of "Carbowax".

As the active ingredient to signal the presence of moisture when insulted with urine, a sufficient amount of wetness indicating agent is used in the composition of the present invention. Useful wetness indicating agents include dye stuffs or colorants and pH indicators which are capable of changing the color of the adhesive composition when insulted with urine or water. Acid - base indicators, which change color in response to a change in pH are preferred and those having a color change at a pH of 2 - 7 are more preferred. This pH is one created by the interaction between moisture and hot melt composition set forth above. Such a pH is created in the hot melt indicator as moisture permeates the hydrophilic organic matrix. The most preferred acid-base indicators include bromophenol blue, bromo chlorophenol blue, bromocresol green and bromocresol purple. Typically they are present in the amounts of 0.01 to 0.5% by weight and the amounts of 0.05% to 0.2% by weight is more preferred. The acid-base indicators herein above described can be purchased from Aldrich Chemical Company Inc. of Milwaukee, WI.

The present invention may include a stabilizer in an amount of from 0% to 2% by weight. Preferably from 0.1% to 1% of a stabilizer is incorporated into the composition. The stabilizers which are useful in .the hot melt wetness indicator adhesive compositions of the present invention are incorporated to help protect the polymers noted above, and thereby the total adhesive system, from the effects of thermal and oxidative degradation which normally occurs during the manufacture and application of the indicator as well as in the ordinary exposure of the final product to the ambient environment. Among the applicable stabilizers are high molecular weight hindered phenols and multifunction phenols, such as sulfur and phosphorous-containing phenols. Hindered phenols are well known to those skilled in the art and may be characterized as phenolic compounds that also contain sterically bulky radicals in close proximity to the phenolic hydroxyl group thereof. In particular, tertiary butyl groups generally are substituted onto the benzene ring in at least one of the ortho positions relative to the phenolic hydroxyl group. Representative hindered phenols include:
1,3,5-trimethyl-2,4,6-tris(3-5-di-tert-butyl-4-hydroxybenzyl) benzene;
pentaerythirtol tetrakis-3(3,5-di-tert-butly-4-hydroxyphenyl) propionate;
n-octadecyl-3(3,5-di-tert-butyl-4-hydroxyphenyl) propionate;
4,4'-methylenebis(4-methyl-6-tert butylphenol);
2,6-di-tert-butylphenol;
6-(4-hydroxyphenoxy)-2,4-bis(n-ocytlthio)-1,3,5-triazine;
2,3,6-tris(4-hydroxy-3,5-di-tert-butyl-phenoxy)-1,3,5-triazine;
di-n-octadecyl-3,5-di-tert-butyl-4-hydroxybenzylphosphonate;
2-(n-octylthio)ethyl-3,5-di-tert-butyl-4-hydroxybenzoate; and
sorbitol hexa-3(3,5-di-tet-butyl-4-hydroxy-phenyl) propionate.

Especially preferred as a stabilizer is pentaerythritol tetrakis-3(3,5-di-tert-butyl-4-hydroxyphenol) propionate.

The performance of these stabilizers may be further enhanced by utilizing, in conjunction therewith; (1) synergists such as, for example, thiodipropionate esters and phosphites; and (2) chelating agents and metal deactivators such as, for example, ethylenediamenetetraacetic acid, salts thereof, and disalicylalpropylenediimine.

It should be understood that other optional additives may be incorporated into the adhesive composition of the present invention in order to modify particular physical properties. These may include, for example, such materials as inert colorants e.g. titanium dioxide, fillers, fluorescent agents, waxes, etc. Typical fillers include talc, calcium carbonate, clay silica, mica, wollastonite, feldspar, aluminum silicate, alumina, hydrated alumina, glass microspheres, ceramic microspheres, thermoplastic microspheres, baryte and wood flour.

The hot melt adhesive composition of the present invention may be formulated using any of the techniques known in the art. A representative example of the prior art mixing procedure involves placing all the components, except the polymer and the pH indicator, in a jacketed mixing kettle equipped with a rotor, and thereafter raising the temperature of the mixture to a range from 121 to 148° C (250 to 300° F) to melt the contents. It shouldbe understood that the precise temperature to be used in this step would depend on the melting points of the particular ingredients. The polymers are subsequently introduced to the kettle under agitation and the mixing is allowed to continue until a consistent and uniform mixture is formed. Finally the acid-base indicator is added and mixing is terminated when the acid-base indicator becomes completely dissolved in the mixture. The contents of the kettle are protected with inert gas such as carbon dioxide and nitrogen during the entire mixing process.

The resulting hot melt wetness indicator may then be applied to substrates using a variety of coating techniques. Examples include hot melt slot die coating, hot melt wheel coating, hot melt roller coating, melt blown coating and spiral spray coating. In a preferred embodiment, the hot melt adhesive is coated onto a substrate using slot-die having 1-5 mm nozzles to produce a coated pattern having multiple wetness indicator stripes on the back sheet.

The adhesive composition of the present invention may be used in a number of nonwoven absorbent articles applications such as, for example, in disposable nonwoven infant and young child diapers, training pants, adult incontinent pad, and briefs, etc.

### TESTS AND MATERIALS

Viscosity was tested according to ASTM D-3236 Method at 121° C (250° F).

Ring & Ball softening point was determined with an automated Herzog unit according to ASTM E-28 Method.

Compatibility was determined by observing the phase behavior of the molten adhesive composition. To carry out the test, 50 grams of molten indicator sample was poured into a 4oz glass jar. The jar containing the sample was then placed in an air-circulating oven at 121°C (250° F). The content of the jar was inspected after 3 days. Phase separation manifested itself by the presence of two distinct layers. The sample was defined as compatible (C) if there was no phase separation, and otherwise it was defined as incompatible (IN).

Rate of color change and color intensity were tested on laminated specimens described in examples 1-7. A strip about 15cm in length was cut off the laminated stock and the release liner was peeled away to expose the coated wetness indicator stripes. The specimen was then insulted with saline solution (Sensitive Eyes® from Bausch & Lomb, Inc.) and the rate of color change was measured with a timer. The time elapsed for the color to change from yellow to blue after the application of saline solution is defined as the rate of color change and is recorded in seconds. The relative intensity of color change was also noted and rated as either poor, fair, good and excellent according to the appearance of the blue image seen through the translucent back sheet on the opposite side of the coated stripes.

Color fastness refers to the ability of the final blue color, obtained after insult, to resist fading. This was measured by using the same specimen for the Rate of Color Change test. After the measurement of Rate of Color Change and Color Intensity, the specimen was subsequently placed with the coated wetness indicator facing downward to a sheet of paper towel that was saturated with the saline solution. It was monitored for 6 hours for color fading. If.the blue color was still clearly visible after the 6 hour period, the product was considered to have passed, otherwise it was considered to have failed.

Environmental stability was tested in a humidity chamber (Envirotronics SH27C, Envirotronics Inc., Grand Rapids, MI.) at 37° C (100° F) and 80% relative humidity. The test specimen was prepared by cutting a strip approximately 5cm.x 10cm off the middle portion of a diaper back sheet and pasting a strip of coated wetness indicator of slightly larger size to cover the cut section in the back sheet. The edges of the wetness indicator coated strips were sealed with 3/4 wide masking tape (Scotch 3M). The specimens were incubated in the humidity chamber described above and the color of the coated wetness indicator was inspected after 48 hours. Environmental stability was assessed as the ability of the specimens to resist premature color change during incubation. A sample was considered to have passed the test if the initial yellow color remain intact, and otherwise it was considered to have failed.
- Escor® AT - 325 is an ethylene-methylacrylate-acrylic acid terpolymer having 20% by weight of methyl acrylate and 6% by weight of acrylic acid. It has a Melt Index (MI) of 20 dg/min measure in accordance with ASTM D1505.
- AC5120 is an ethylene-acrylic acid copolymer wax having an acid number of 120 mg KOH/g. It is commercially available from Honeywell Specialty Chemicals, Inc. of Morristown, NJ.
- Elvax® 410, purchased from Dupont Company, Inc. of Wilmington DE, is an ethylene-vinyl acetate (EVA) copolymer having 28% by weight of vinyl acetate and 420 dg/min Melt Index (MI).
- Sylvaros® PR295 is a polymerized tall oil rosin having an acid number of 162 mg KOH/g and a softening point of approximately 96 °C. It is purchased from Arizona Chemical Company of Jacksonville, FL.
- Sylvarez TP2040 is a terpene - phenolic resin having a softening point of 118 °C. It is also purchased from Arizona Chemical Company.
- Starplex® 90K, obtained from American Ingredients Co., Kansas City, MO., is a distilled monoglyceride non-ionic surfactant with a HLB value of 3.0.
- Genapol ® T800, purchases from Clairant Corporation, Charlotte, N.C., is a ethoxylated fatty alcohol non-ionic with a HLB value of 12.
- Span® 60 is a sorbitan monostearate surfactant with a HLB value of 4.7. It is commercially available through Uniqema Inc., Wilmington, DE.
- Atmer® 290 is a glycerol monostearate surfactant having a HLB value of 3.4. It is also purchased from Uniqema Inc.
- Aerosol® OT-100 is a dioctyl sodium sulfosuccinate anionic surfactant. It is commercially available through Cytec Industries, Inc., Morristown, NJ.
- Lonzest® MSA is a surfactant (HLB = 11) containing a mixture of glycerol monostearate and citric acid. It is commercially available from Lonza Inc., Fair Lawn, NJ.
- Bromonphenol blue and bromocresol green acid - base indicators are reagent grade products purchased from Aldrich Chemical Company, Milwaukee, WI.
- Isostearic acid is also purchases from Aldrich Chemical Company.

The invention is further illustrated by way of the examples set forth below.

### EXAMPLES 1 - 2

Hot melt wetness indicating adhesives of Examples 1 - 2 were prepared with the ingredients shown in Table 1. A total of 250 grams each were made and the mixing was carried out at 121°C (250°F) under carbon dioxide (CO₂) atmosphere in a laboratory propeller type mixer, which consisted of a propeller powered by a motor, a heating mantle, a temperature control unit and a container of 1 quart in size.
The appropriate amount of each components, calculated according to the ratios shown in Table 1, except the polymer and the pH indicators, were charged to the container. The container was then heated to melt the contents. After the components were completely melted, the motor was turned on to start stirring. The polymer was next added to the mixer and mixing was continued until the polymer was dissolved. The pH indicators were introduced last and the contents stirred for an additional two-hour period. The final mixtures exhibit yellow color in dry state and the color turns from yellow to blue after they are insulted with body exudates, such as urine. The mixtures of Examples 1 - 2 are useful as hot melt wetness indicator adhesives for disposable nonwoven absorbent article applications. When applied, for example, to the backsheet of baby diaper, these products serve as a signaling mechanism to indicate the presence of body exudates after insult.

Several tests were carried out according to the procedures described in Material and Testing of the present invention. Specimens for color change tests were prepared by slot die coating onto a 1 mil. thick white polyethylene film substrate by using a bench scale hot melt coater (Acumeter Model LH-1) The slot die had three nozzles of equal width (2 mm) to obtain a three-stripe coated pattern. The coating weight was controlled at 0.15 g/m/line. The coated polyethylene film was laminated online to release paper to prevent it from blocking. Prior to testing, the release paper was peeled off from the coated polyethylene substrate to expose the wetness indicator. The test results are shown in Table 1.

**TABLE 1**

| . EXAMPLES 1 - 2 | | |
|---|---|---|
| Components | Percent (%) by Weight | |
| | 1 | 2 |
| Escor® AT 325 | 25.0 | 20.0 |
| Sylvarez TP 2040 | 39.4 | - |
| Sylvaros® PR 295 | - | 25.4 |
| Starplex® 90K | 35.0 | 50.0 |
| Genapol® T-800 | - | 4.0 |
| Antioxidant | 0.5 | 0.5 |
| Bromophenol Blue | 0.05 | 0.10 |
| Bromocresol Green | 0.10 | - |
| D&C Orange #4 | - | 0.01 |
| | | |
| Viscosity (cP) @ 121°C (250°F) | 11500 | 1860 |
| Softening Point °C (°F) | 61 (143) | 72 (162) |
| Compatibility | C | C |
| Dry Color | Yellow | Yellow |
| Color after Insult | Blue | Blue |
| Rate of Color Change (h) | 4 | 0.3 |
| Intensity | Good | Good |
| Fastness | Pass | Pass |
| Environmental Stability | Pass | Pass |

As shown in Table 1, the wetness indicators of Examples 1 - 2 are formulated to have delayed color change and can withstand multiple insults. Such products are advantageous for baby diaper applications where caregivers usually do not want to change the diaper after only a single insult.

### EXAMPLES 3 - 7

Hot melt wetness indicators of Examples 3 - 7 are formulated to have fast color change and are advantageous for adult incontinent product applications where change is required immediately after insult by the wearer. These examples were prepared by using

**TABLE 2.**

| EXAMPLES 3 - 7 | | | | | |
|---|---|---|---|---|---|
| Components | Percent (%) by Weight | | | | |
| | 3 | 4 | 5 | 6 | 7 |
| Escor® AT 325 | 20.0 | 20.0 | 20.0 | 20.0 | 15.0 |
| Sylvaros® PR 295 | 47.4 | 37.4 | 42.4 | 37.4 | 34.4 |
| Starplex® 90 K | 20.0 | 30.0 | 25.0 | 25.0 | - |
| Atmer® 129 | - | - | - | - | 25.0 |
| Genapol® T-800 | 12.0 | 12.0 | - | 12.0 | - |
| Lonzest® MSA | - | - | - | - | 25.0 |
| Span® 60 | - | - | 30.0 | - | - |
| Benzoflex 9-88 | - | - | - | 5.0 | - |
| Antioxidant | 0.5 | 0.5 | 0.5 | 0.5 | 0.5 |
| Bromophenol Blue | 0.05 | 0.05 | 0.05 | 0.05 | - |
| Bromocresol Green | 0.10 | 0.10 | 0.10 | 0.10 | 0.15 |
| | | | | | |
| Viscosity (cP) @121 °C (250 °F) | 1310 | 1880 | 3710 | 1010 | |
| Softening Point °C (°F) | 75 (168) | 75 (167) | 76 (170) | 75 (168) | |
| Compatibility | C | C | C | C | C |
| Dry Color | Yellow | Yellow | Yellow | Yellow | Yellow |
| Color after Insult | Blue | Blue | Blue | Blue | Blue |
| Rate of Color Change (sec) | 120 | 120 | 60 | 30 | 70 |
| Intensity | Excellent | Excellent | Excellent | Excellent | Excellent |
| Fastness | Pass | Pass | Pass | Pass | Pass |
| Environmental Stability | Pass | Pass | Pass | Pass | Pass |

the ingredients shown in Table 2. Mixing, specimen preparation and test were done by using the same method herein described above in Examples 1 - 2. The test results are also listed in Table 2. The wetness indicators of Examples of 3 - 7 all have yellow dry color, which changes to blue upon insult with urine. They exhibit excellent compatibility, quick color change, high color intensity, and good color fastness and good environmental stability.

### COMPARATIVE EXAMPLES 8 - 10

Comparative Examples 8 - 10 were prepared in accordance to the prior art compositions disclosed by Colon et al. in US Patent 4,681,576, 4,743,238 and 4,895,567. The compositions of Comparative Examples 8 - 10 correspond, respectively, to Examples 9, 22 and 23 of the '567 patent. Comparative Example 8 represents a composition consisting of a blend of water soluble vinyl pyrrolidone-vinyl acetate copolymer (Luviskol® VA-64) and water insoluble ethylene-vinyl acetate copolymer; Comparative Examples 9 and 10 represent compositions based on a low molecular weight ethylene-acrylic acid polymer wax (AC-5120 Allied Signal) as the major component. The ingredients and their amounts for each comparative example are shown in Table 3 along with physical and performance data. As shown in the table, Comparative Examples 8 and 10, when molten, became phase separated overnight, indicating that the components are incompatible. Because phase separation could start in the melting tank of a hot melt coater during the specimen preparation process, performance tests were not carried out on Comparative Examples 8 and 10. Due to their incompatibility, these two comparative examples are virtually useless as hot melt for disposable absorbent article applications. Comparative Example 9, on the other hand, did not show a color change when tested according to the method herein described in Example 1 of the present invention. As a consequence, color intensity, fastness and environmental stability tests could also not be carried out. Such a product, once again, will have no practical value as a wetness indicator.

**TABLE 3**

| COMPARATIVE EXAMPLES 8 - 10 | | | |
|---|---|---|---|
| Component | Percent (%) by Weight | | |
| | 8 | 9 | 10 |
| Luviskol® VA-64 | 36.3 | - | - |
| Elvax® 410 | 18.2 | - | 8.0 |
| AC 5120 | - | 80.0 | 65.0 |
| Isostearic Acid | 45.3 | - | 5.0 |
| Aerosol® OT-100 | - | 20.0 | 22.0 |
| Antioxidant | 0.1 | 0.3 | 0.3 |
| Bromophenol Blue | 0.07 | 0.075 | 0.075 |
| | | | |
| Viscosity (cP) @ 121°C (250°F) | 2910 | 1240 | 1050 |
| Softening Point °C (°F) | 82 (181) | 77 (172) | 73 (165) |
| Compatibility | Incompatible | Compatible | Incompatible |
| Dry Color | Green/blue | Pale yellow | Pale yellow |
| Color after insult | N/A | No change | N/A |
| Rate of Change (s) | N/A | No change | N/A |

## Claims

1. A hot melt wetness indicating adhesive composition, comprising a blend of the following ingredients:
10% to 40% by weight of an ethylene-alkylacrylate-acrylic acid terpolymer;
20% to 60% by weight of a polar tackifier;
15% to 60% by weight of a surfactant or blend of surfactants;
0% to 20% by weight of a plasticizer;
0% to 2% by weight of a stabilizer; and
0.01% to 0.5% by weight of a wetness indicator;
the ingredients totalling 100% by weight of the composition.

2. The composition of claim 1 wherein the said terpolymer is an ethylene-methylacrylate-acrylic acid terpolymer containing from 5% to 35% by weight of methyl acrylate and from 2% to 25% by weight acrylic acid.

3. The composition of claim 1 wherein said terpolymer has a melt index value greater than 5g/10 min.

4. The composition of claim 1 wherein the said tackifier is selected from the group consisting of rosin, modified rosin, hydrogenated rosin, rosin ester and terpene-phenolic resin.

5. The composition of claim 4 wherein the said modified rosin is a polymerized rosin having an acid number between 100 and 170mg KOH/g and a softening point greater than 80°C.

6. The composition of claim 1 wherein the said surfactant is a non-ionic surfactant having a HLB value less than 20.

7. The composition of claim 1 wherein said surfactant has a HLB value less than 15.

8. The composition of claim 1 wherein said surfactant has a HLB value between 3 and 12.

9. The composition of claim 6 where the said nonionic surfactant is selected from the group consisting of alkyl amines and amides, alkanoamines and amides, amine oxides, ethoxylated fatty alcohol, ethoxylated fatty acids, ethoxylated alkylphenols, ethoxylated amines and amides, ethoxylated fatty esters and oils, glycerides and their derivatives, sorbitan derivatives, sucrose and glucose derivatives.

10. The composition of claim 1 wherein the said surfactant is an ethoxylated fatty alcohol.

11. The composition of claim 1 wherein the said surfactant is a mono- or di- fatty ester of glycerol.

12. The composition of claim 1 wherein the said plasticizer is selected from the group consisting of liquid polyethylene glycol and a dibenzoate ester.

13. The composition of claim 12 wherein said dibenzoate ester is diethylene glycol dibenzoate.

14. The composition of claim 1 wherein said wetness indicator is a pH indicator and comprises bromophenol blue.

15. The composition of claim 1 wherein said wetness indicator is a pH indicator and comprises bromocresol green.

16. The composition of claim 1 wherein said wetness indicator is a pH indicator and comprises a mixture of bromophenol blue and bromocresol green.

17. The composition of claim 1 wherein the said composition further includes an inert dye or colorant.

18. The composition of claim 1 wherein the said composition further includes a fluorescing agent

19. The composition of claim 1 wherein the said composition further includes a filler selected from the group consisting of talc, calcium carbonate, clay silica, mica, wollastonite, feldspar, aluminum silicate, alumina, hydrated alumina, glass microspheres, ceramic microspheres, thermoplastic microspheres, baryte, waxes and wood flour.

20. The composition of claim 1 containing 15% to 30% by weight of said terpolymer.

21. The composition of claim 1 containing 25% to 50% by weight of said tackifier.

22. The composition of claim 1 containing 25% to 45% by weight of said surfactant or blend of surfactants.

23. The composition of claim 1 containing 5% to 15% by weight of said plasticizer.

24. The composition of claim 1 containing 0.1% to 1% by weight of said stabilizer.

25. The composition of claim 1 containing 0.05% to 0.2% by weight of said wetness indicator.

26. A disposable nonwoven absorbent article coated at least in part with a composition of claim 1 applied thereto as a hot melt adhesive.

## Patentansprüche

1. Heißschmelzklebstoffzusammensetzung mit Nässeanzeigung, die ein Gemisch der folgenden Bestandteile umfasst:
| | |
|---|---|
| 10 bis 40 Gew.-% | Ethylen/Alkylacrylat/Acrylsäure-Terpolymer, |
| 20 bis 60 Gew.-% | polaren Klebrigmacher, |
| 15 bis 60 Gew.-% | Tensid oder Tensidgemisch, |
| 0 bis 20 Gew.-% | Weichmacher, |
| 0 bis 2 Gew.-% | Stabilisator und |
| 0,01 bis 0,5 Gew.-% | Nässeindikator, |
wobei die Bestandteile insgesamt 100 Gew.-% der Zusammensetzung ausmachen.

2. Zusammensetzung nach Anspruch 1, bei der das Terpolymer ein Ethylen/Methylacrylat/Acrylsäure-Terpolymer ist, das 5 bis 35 Gew.-% Methacrylat und 2 bis 25 Gew.-% Acrylsäure enthält.

3. Zusammensetzung nach Anspruch 1, bei der das Terpolymer einen Schmelzindexwert größer als 5 g/10 min aufweist.

4. Zusammensetzung nach Anspruch 1, bei der der Klebrigmacher ausgewählt ist aus der Gruppe bestehend aus Rosinharz, modifiziertem Rosinharz, hydriertem Rosinharz, Rosinharzester und Terpen/Phenol-Harz.

5. Zusammensetzung nach Anspruch 4, bei der das modifizierte Rosinharz ein polymerisiertes Rosinharz mit einer Säurezahl zwischen 100 und 170 mg KOH/g und einem Erweichungspunkt oberhalb von 80 °C ist.

6. Zusammensetzung nach Anspruch 1, bei der das Tensid ein nichtionisches Tensid mit einem HLB-Wert unterhalb von 20 ist.

7. Zusammensetzung nach Anspruch 1, bei der das Tensid einen HLB-Wert unterhalb von 15 aufweist.

8. Zusammensetzung nach Anspruch 1, bei der das Tensid einen HLB-Wert zwischen 3 und 12 aufweist.

9. Zusammensetzung nach Anspruch 6, bei der das nichtionische Tensid ausgewählt ist aus der Gruppe bestehend aus Alkylaminen und -amiden, Alkanolaminen und -amiden, Aminoxiden, ethoxyliertem Fettalkohol, ethoxylierten Fettsäuren, ethoxylierten Alkylphenolen, ethoxylierten Aminen und Amiden, ethoxylierten Fettestern und ölen, Glyceriden und deren Derivaten, Sorbitanderivaten, Sucrose- und Glucosederivaten.

10. Zusammensetzung nach Anspruch 1, bei der das Tensid ein ethoxylierter Fettalkohol ist.

11. Zusammensetzung nach Anspruch 1, bei der das Tensid ein Mono- oder Difettester von Glycerin ist.

12. Zusammensetzung nach Anspruch 1, bei der der Weichmacher ausgewählt ist aus der Gruppe bestehend aus flüssigem Polyethylenglykol und Dibenzoatester.

13. Zusammensetzung nach Anspruch 12, bei der der Dibenzoatester Diethylenglykoldibenzoat ist.

14. Zusammensetzung nach Anspruch 1, bei der der Nässeindikator ein pH-Indikator ist und Bromphenolblau umfasst.

15. Zusammensetzung nach Anspruch 1, bei der der Nässeindikator ein pH-Indikator ist und Bromkresolgrün umfasst.

16. Zusammensetzung nach Anspruch 1, bei der der Nässeindikator ein pH-Indikator ist und eine Mischung aus Bromphenolblau und Bromkresolgrün umfasst.

17. Zusammensetzung nach Anspruch 1, bei der die Zusammensetzung ferner einen inerten Farbstoff oder ein inertes Färbemittel enthält.

18. Zusammensetzung nach Anspruch 1, bei der die Zusammensetzung ferner ein fluoreszierendes Mittel enthält.

19. Zusammensetzung nach Anspruch 1, bei der die Zusammensetzung ferner einen Füllstoff enthält, der ausgewählt ist aus der Gruppe bestehend aus Talk, Calciumcarbonat, Ton, Siliciumdioxid, Mica, Wollastonit, Feldspat, Aluminiumsilikat, Aluminiumoxid, hydratisiertem Aluminiumoxid, Glasmikrokugeln, keramischen Mikrokugeln, thermoplastischen Mikrokugeln, Baryt, Wachsen und Holzmehl.

20. Zusammensetzung nach Anspruch 1, die 15 bis 30 Gew.-% des Terpolymers enthält.

21. Zusammensetzung nach Anspruch 1, die 25 bis 50 Gew.-% des Klebrigmachers enthält.

22. Zusammensetzung nach Anspruch 1, die 25 bis 45 Gew.-% des Tensids oder eines Gemisches von Tensiden enthält.

23. Zusammensetzung nach Anspruch 1, die 5 bis 15 Gew.-% des Weichmachers enthält.

24. Zusammensetzung nach Anspruch 1, die 0,1 bis 1 Gew.-% des Stabilisators enthält.

25. Zusammensetzung nach Anspruch 1, die 0,05 bis 0,2 Gew.-% des Nässeindikators enthält.

26. Adsorbierender Einwegvliesgegenstand, der zumindest teilweise mit einer Zusammensetzung gemäß Anspruch 1 beschichtet ist, die darauf als Heißschmelzklebstoff aufgebracht worden ist.

## Revendications

1. Une composition thermofusible adhésive indicatrice d'humidité, comprenant :
de 10% à 40% en poids d'un terpolymère éthylène-acrylate d'alkyle-acide acrylique ;
de 20% à 60% en poids d'un agent collant polaire ;
de 15% à 60% en poids d'un tensioactif ou d'un mélange de tensioactifs ;
de 0% à 20% en poids d'un agent plastifiant ;
de 0% à 2% en poids d'un stabilisateur ; et
de 0,01% à 0,5% en poids d'un indicateur d'humidité ;
les ingrédients totalisant 100% en poids de la composition.

2. La composition selon la revendication 1, dans laquelle ledit terpolymère est un terpolymère éthylène-acrylate de méthyle-acide acrylique contenant de 5% à 35% en poids d'acrylate de méthyle et de 2% à 25% en poids d'acide acrylique.

3. La composition selon la revendication 1, dans laquelle ledit terpolymère présente une valeur d'indice de fusion supérieure à 5g/10min.

4. La composition selon la revendication 1, dans laquelle ledit agent collant est choisi dans le groupe constitué de colophane, colophane modifiée, colophane hydrogénée, ester de colophane et d'une résine terpène phénolique.

5. La composition selon la revendication 4, dans laquelle ladite colophane modifiée est une colophane polymérisée ayant un nombre d'acide compris entre 100 et 170mg de KOH/g et un point de ramollissement supérieur à 80°C.

6. La composition selon la revendication 1, dans laquelle ledit tensioactif est un tensioactif non ionique ayant une valeur de BHL (balance hydrophile-lipophile) inférieure à 20.

7. La composition selon la revendication 1, dans laquelle ledit tensioactif possède une valeur de BHL inférieure à 15.

8. La composition selon la revendication 1, dans laquelle ledit tensioactif a une valeur de BHL comprise entre 3 et 12.

9. La composition selon la revendication 6, dans laquelle ledit tensioactif non ionique est choisi dans le groupe constitué par les amines et les amides alkylées, les alkanoamines et amides, les oxydes d'amines, les alcools gras éthoxylés, les acides gras éthoxylés, les alkylphénols éthoxylés, les amines et les amides éthoxylées, les esters et huiles gras éthoxylés, les glycérides et leurs dérivés, les dérivés de sorbitane et les dérivés de saccharose et de glucose.

10. La composition selon la revendication 1, dans laquelle ledit tensioactif est un alcool gras éthoxylé.

11. La composition selon la revendication 1, dans laquelle ledit tensioactif est un mono- ou di-ester gras de glycérol.

12. La composition selon la revendication 1, dans laquelle ledit agent plasifiant est choisi dans le groupe constitué de glycol de polyéthylène et d'ester dibenzoate liquides.

13. La composition selon la revendication 12, dans laquelle ledit ester dibenzoate est le dibenzoate de diéthylène glycol.

14. La composition selon la revendication 1, dans laquelle ledit indicateur d'humidité est un indicateur de pH et comprend du bleu de bromophénol.

15. La composition selon la revendication 1, dans laquelle ledit indicateur d'humidité est un indicateur de pH et comprend du vert de bromocrésol.

16. La composition selon la revendication 1, dans laquelle ledit indicateur d'humidité est un indicateur de pH et comprend un mélange de bleu de bromophénol et de vert de bromocrésol.

17. La composition selon la revendication 1, **caractérisée en ce qu'**elle comprend en outre une teinture ou un colorant inerte.

18. La composition selon la revendication 1, **caractérisée en ce qu'**elle comprend en outre un agent fluorescent.

19. La composition selon la revendication 1, **caractérisée en ce qu'**elle comprend en outre une charge choisie dans le groupe constitué de talc, de carbonate de calcium, de silice d'argile, de mica, de wollastonite, de felspar, de silicate d'aluminium, d'alumine, d'alumine hydratée, de microsphères de verre, de microsphères de céramique, de microsphères thermoplastiques, de baryte, de cires et farine de bois.

20. La composition selon la revendication 1 contenant 15% à 30% en poids dudit terpolymère.

21. La composition selon la revendication 1 contenant 25% à 50% en poids dudit agent collant.

22. La composition selon la revendication 1 contenant 25% à 45% en poids dudit tensioactif ou mélange de tensioactifs.

23. La composition selon la revendication 1 contenant 5% à 15% en poids dudit agent plastifiant.

24. La composition selon la revendication 1 contenant 0,1% à 1% en poids dudit stabilisateur.

25. La composition selon la revendication 1 contenant 0,05% à 0,2% en poids dudit indicateur d'humidité.

26. Un article absorbant non tissé jetable revêtu au moins en partie avec une composition selon la revendication 1 appliquée dessus en tant qu'adhésif thermofusible.
